(19) Europäisches Patentamt
European Patent Office
Office européen des brevets

(11) Veröffentlichungsnummer: **0 037 030**
**A1**

# (12) EUROPÄISCHE PATENTANMELDUNG

(21) Anmeldenummer: **81102091.6**

(22) Anmeldetag: **20.03.81**

(51) Int. Cl.³: **C 07 C 143/80**
//C08G18/38

(30) Priorität: **02.04.80 DE 3012800**

(43) Veröffentlichungstag der Anmeldung:
**07.10.81 Patentblatt 81/40**

(84) Benannte Vertragsstaaten:
**BE DE FR GB IT**

(71) Anmelder: **BAYER AG**
**Zentralbereich Patente, Marken und Lizenzen**
**D-5090 Leverkusen 1, Bayerwerk(DE)**

(72) Erfinder: **Kopp, Richard, Dr.**
**Wolfskaul 12**
**D-5000 Köln 80(DE)**

(72) Erfinder: **Meyborg, Holger, Dr.**
**Bergisch-Gladbacher-Strasse 1**
**D-5068 Odenthal(DE)**

(72) Erfinder: **Grögler, Gerhard, Dr.**
**von-Diergardt-Strasse 46**
**D-5090 Leverkusen 1(DE)**

(72) Erfinder: **Schwindt, Jürgen, Dr.**
**Heinrich-von-Kleist-Platz 4**
**D-5090 Leverkusen 1(DE)**

## (54) Sulfonamidgruppenhaltige aromatische Diamine.

(57) Die Erfindung betrifft neue sulfonamid-gruppenhaltige aromatische Diamine der Formel

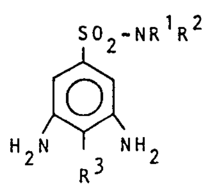

in der

$R^1$ und $R^2$ gleich oder verschieden sind und einen gegebenenfalls verzweigten Alkylrest mit 1 bis 20 C-Atomen oder einen Aryl-oder Aralkylrest mit 6 bis 20 C-Atomen bedeuten und

$R^3$ für ein Wasserstoffatom, einen gegebenenfalls verzweigten Alkylrest mit 1 bis 6 C-Atomen, einen Alkoxyrest mit 1 bis 6 C-Atomen oder ein Halogen-atom steht.

EP 0 037 030 A1

Croydon Printing Company Ltd.

0037030

BAYER AKTIENGESELLSCHAFT 5090 Leverkusen, Bayerwerk
Zentralbereich
Patente, Marken und Lizenzen Sft/Kü-c

## Sulfonamidgruppenhaltige aromatische Diamine

Die vorliegende Erfindung betrifft neue sulfonamidgruppenhaltige aromatische Diamine der allgemeinen Formel (I)

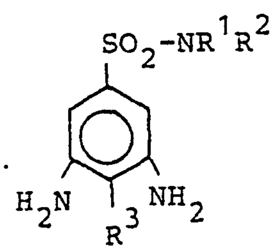

(I)

in der

$R^1$ und $R^2$ gleich oder verschieden sind und einen gegebenenfalls verzweigten Alkylrest mit 1 bis 20 ($R^1$ vorzugsweise 1 bis 6 C-Atomen), oder einen Aryl- oder Aralkylrest mit 6 bis 20, vorzugsweise 6 bis 9 C-Atomen und

$R^3$ ein Wasserstoffatom, einen gegebenenfalls verzweigten Alkylrest mit 1 bis 6 C-Atomen, vorzugsweise eine Methylgruppe, einen Alkoxyrest mit 1 bis 6 C-Atomen oder ein Halogenatom (vorzugsweise Chlor) bedeuten.

Die Herstellung der neuen Verbindungen ist einfach und geschieht in der Weise, daß man sekundäre Amine der allgemeinen Formel (II) oder deren Hydrochloride der allgemeinen Formel (III), in der $R^1$ und $R^2$ gemäß Formel (I) definiert sind, in Gegenwart von

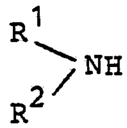

(II)

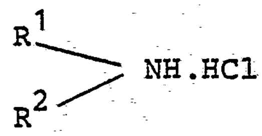

(III)

alkalisch reagierenden Verbindungen mit aromatischen Sulfochloriden der allgemeinen Formel (IV) umsetzt, in der $R^3$ gemäß Formel (I) definiert ist.

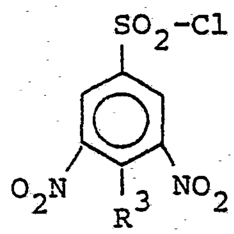

(IV)

Die dabei erhaltenen Dinitroverbindungen der allgemeinen Formel (V)

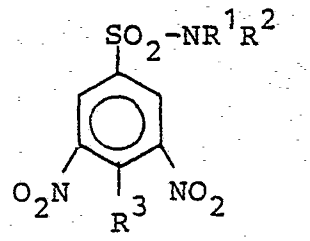

(V)

können anschließend in an sich bekannter Weise, z.B. durch katalytische Hydrierung, zu den erfindungsgemäßen Diaminen (I) reduziert werden.

Als sekundäre Amine der Formel (II) bzw. deren Hydrochloride (III) zur Herstellung der erfindungsgemäßen Verbindungen seien beispielsweise Dimethylamin Diethylamin, Di-n-propylamin, Diisopropylamin, Di-n-butylamin, Diisobutylamin, Di-isoamylamin, Di-n-hexylamin, Bis(2-ethylhexylamin), Dioctylamin, Distearylamin und N-Methylstearylamin bzw. die entsprechenden Hydrochloride genannt.

Weitere Ausgangsprodukte sind die 3,5-Dinitrobenzolsulfonylchloride (IV), vorzugsweise das 3,5-Dinitro-4-chlorbenzolsulfonylchlorid und 2,6-Dinitrotoluol-4-sulfonylchlorid. Diese Ausgangsverbindungen sind in an sich bekannter Weise durch Chlorierung der entsprechenden Benzolsulfonsäure-Alkalisalze zugänglich.

Als alkalisch reagierende Verbindungen, die zur Umsetzung der Amine (II) mit den Dinitro-Benzolsulfonylchloriden (IV) erforderlich sind, sind z.B. Erdalkali- und Alkalihydroxide oder auch eines der vorgenannten Amine der Formel (II), welches dann im molaren Überschuß eingesetzt werden muß, oder tertiäre Amine wie z.B. Pyridin oder Triethylamin geeignet.

Werden Aminhydrochloride der Formel (III)

als Aminkomponente eingesetzt, so kommen als alkalisch reagierende Verbindungen nur Erdalkali- oder Alkalihydroxide in Betracht.

Zur Sulfonamidbildung kann das Molverhältnis der beiden Komponenten Dinitrobenzolsulfochlorid (IV) und Amin (II) bzw. Hydrochlorid (III) zwischen 1 : 2,5 und 1 : 1 liegen, bevorzugt setzt man jedoch beide Stoffe etwa äquimolar ein. Der bei der Reaktion frei werdende Chlorwasserstoff kann, wie schon erwähnt, entweder durch überschüssiges Amin (II) oder durch den Zusatz einer weiteren Base, z.B. eines tertiären Amins oder von Alkali- oder Erdalkalihydroxid gebunden werden.

Die Umsetzung kann durch gleichzeitiges Zusammenmischen aller drei Komponenten oder aber durch Vorlegen einer bzw. zweier Komponenten und anschließende Zugabe der beiden übrigen bzw. der dritten Komponente vorgenommen werden. So kann man beispielsweise das Amin vorlegen und das Dinitrobenzolsulfochlorid und die alkalisch reagierende Verbindung gleichzeitig getrennt zulaufen lassen. Die besten Ausbeuten erhält man bei der Umsetzung etwa äquimolarer Mengen Sulfochlorid und Amin jedoch, wenn man das Sulfochlorid (IV) in einem polaren organischen Lösungsmittel suspendiert vorlegt und das Amin und die Base gleichzeitig zulaufen läßt.

Als Reaktionsmedium kommen dabei Wasser oder auch organische Lösungsmittel in Frage; die Reaktionspartner können in homogener Phase oder zweiphasig, gelöst, emulgiert oder suspendiert vorliegen, wobei jedoch das Dinitrobenzolsulfochlorid (IV) vorzugsweise in einem polaren Lösungsmittel gelöst oder suspendiert eingesetzt wird. Die Sulfonamidbildung verläuft im gesamten alkalischen Bereich bei Temperaturen von -20°C bis 60°C in guten bis zufriedenstellenden Ausbeuten.

Das Dinitrobenzolsulfonamid der Formel (V) kann dann nach an sich bekannten Verfahren reduziert werden.

Bevorzugt wird die in einem inerten Lösungsmittel gelöste Dinitroverbindung unter Druck in Gegenwart von üblichen Hydrierungskatalysatoren hydriert.

Geeignete Lösungsmittel für Dinitrobenzolsulfochlorid (IV) und Dinitrobenzolsulfonamid (V) sind beispielsweise: Toluol, Tetrahydrofuran, Dioxan, Ethanol, Aceton, Butanon, Dimethylformamid und Essigester.

Beispiele für die erfindungsgemäßen Diamine sind die folgenden Verbindungen:

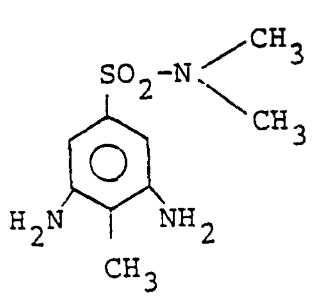

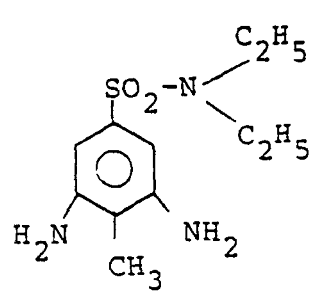

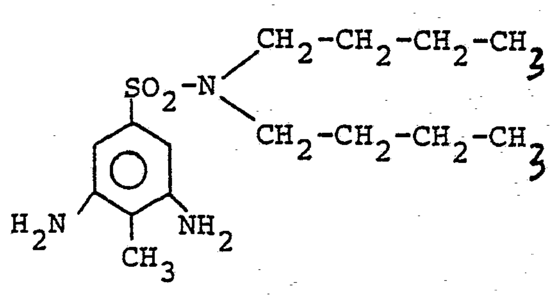

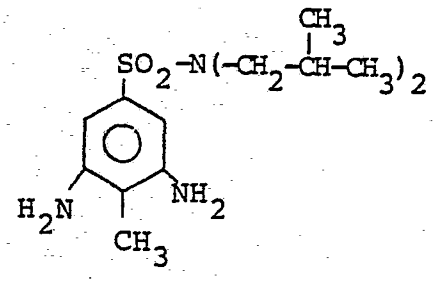

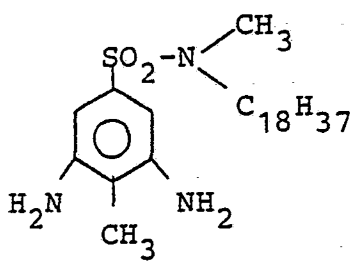

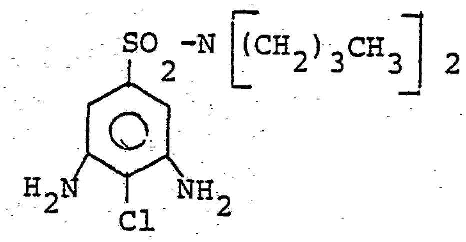

Die neuen Verbindungen sind in der Regel gut kristallisiert und können z.B. als Ausgangsmaterialien zur Herstellung von Farbstoffen, Pflanzenschutzmitteln und Kunststoffen (Polyurethane, Polyamide, Epoxide u.ä.) dienen. Als besonders wertvoll erwiesen sich die Verbindungen als Kettenverlängerungsmittel bei der Herstellung von Kunststoffen mit kautschukelastischen Eigenschaften nach dem bekannten Isocyanat-Polyadditionsverfahren.

Die folgenden Beispiele sollen die Herstellung der erfindungsgemäßen Verbindungen erläutern. Wo nicht anders vermerkt, sind Mengenangaben als Gewichtsteile bzw. Gewichtsprozente zu verstehen.

Beispiel 1

a) Herstellung von 2,6-Dinitrotoluol-4-sulfochlorid

182 g trockenes Na-Salz der 2,6-Dinitrotoluol-4-sulfonsäure werden mit 225 ml Thionylchlorid und 5,1 ml Dimethylformamid 30 Minuten unter Rückfluß erhitzt. Danach wird das Thionylchlorid abdestilliert (Reste werden durch das Hindurchleiten eines Stickstoffstromes entfernt) und der Rückstand in Eiswasser gegeben. Das ausfallende Sulfochlorid wird abfiltriert, mehrmals mit Wasser gewaschen und im Vakuumtrockenschrank bei 50°C getrocknet.
Man erhält ein hellbraunes Pulver mit einem Fp. von 126 - 128°C.
Ausbeute: 85 % der Theorie.

b) Herstellung von 2,6-Dinitrotoluol-4-N,N-di-n-butylsulfonamid

Zu einer Suspension von 30,9 g (0,11 Mol) 2,6-Dinitrotoluol-4-sulfochlorid in 300 ml Dioxan wird bei 10°C eine Lösung von 12,9 g (0,1 Mol) Dibutylamin und 11,1 g (0,11 Mol) Triethylamin in 100 ml Dioxan gegeben. Die Reaktionsmischung wird 2 Stunden bei 50°C gerührt. Der graue Niederschlag (Triethylaminhydrochlorid: Fp.: 253-255°C) wird abfiltriert und mit Dioxan gewaschen. Die Dioxanphasen werden am Rotationsverdampfer zur Trockne gebracht.
Hellbraunes Pulver (Fp.: 85-87°C)
Ausbeute: 98 % der Theorie.
Reinigung durch Umkristallisieren aus Methanol (Fp.: 94-95°C)

| Elementaranalyse: | | ber. | gef. |
|---|---|---|---|
| | C | 48,26 | 48,2 |
| | H | 6,17 | 6,3 |
| | N | 11,26 | 11,2 |
| | O | 25,74 | |
| | S | 8,58 | |

c) Herstellung von 2,6-Diaminotoluol-4-N,N-di-n-butylsulfonamid

329 g der Dinitroverbindung werden in 2 l Ethanol gelöst und in Gegenwart von 60 g Raney-Nickel unter Druck hydriert. Das Raney-Nickel wird abfiltriert und die Lösung im Vakuum eingeengt. Der Rückstand wird bei 60°C im Vakuumtrockenschrank getrocknet.
Grau-braunes Pulver (Fp.: 121 - 123°C).
Rohausbeute: 96 %.
Durch zweimaliges Erhitzen des Produktes mit Ethanol/Wasser (1:1) werden die Verunreinigungen herausgelöst.
Weißes Pulver (Fp.: 124°C).
Reinausbeute bei der Hydrierung: 73 % der Theorie.

| Elementaranalyse: | | ber. | gef. |
|---|---|---|---|
| | C | 57,51 | 57,6 |
| | H | 8,63 | 8,8 |
| | N | 13,42 | 13,4 |

| Elementaranalyse: | | ber. | gef. |
|---|---|---|---|
| | O | 10,22 | |
| | S | 10,22 | 10,2 |

Beispiel 2

a) Herstellung von 2,6-Dinitrotoluol-4-(N-methyl-N-stearyl)sulfonamid

Zu einer Suspension von 30,9 g (0,11 Mol) 2,6-Dinitrotoluol-4-sulfochlorid in 300 ml Toluol wird bei ca. 10°C eine Lösung von 28,3 g (0,1 Mol) N-Methylstearylamin und 11,1 g (0,11 Mol) Triethylamin in 200 ml Toluol gegeben. Die Reaktionsmischung wird 2 Stunden bei 50°C gerührt. Der graue Niederschlag (Triethylaminhydrochlorid Fp.: 248-250°C) wird abfiltriert und mit Toluol gewaschen. Die Toluolphasen werden am Rotationsverdampfer zur Trockne eingeengt.
Hellbraunes Pulver (Fp.: 92-94°C)
Ausbeute: 99 % der Theorie.

b) Herstellung von 2,6-Diaminotoluol-4-(N-methyl-N-stearyl)sulfonamid

64 g 2,6-Dinitrotoluol-4-(N-methyl-N-stearyl)sulfonamid werden in 530 ml Ethanol gelöst und in Gegenwart von 15 g Raney-Nickel unter Druck hydriert. Nach der Umsetzung wird das Raney-Nickel abfiltriert.

das Filtrat eingeengt, der Rückstand mehrmals mit Wasser gewaschen und getrocknet.
Grau-braunes Pulver (Fp.: 77 - 79°C).
Ausbeute: 75 % der Theorie.

Beispiel 3

Zu einer Suspension von 28,1 g (0,1 Mol) 2,6-Dinitrotoluol-4-sulfochlorid in 150 ml Ethanol wird bei ca. 20°C eine Lösung von 15,3 g (0,21 Mol) Diethylamin in 50 ml Ethanol gegeben. Die Reaktionsmischung wird 2 Stunden bei 50°C gerührt. Dann werden 500 ml Wasser zugegeben und der entstehende Niederschlag wird abgesaugt, mit Wasser gewaschen und getrocknet.
Hellbraunes Pulver (Fp.: 108-110°C)
Ausbeute: 60 % der Theorie.

Die Dinitroverbindung wird analog zu Beispiel 2b) zum Diamin hydriert.
Rosa-braunes Pulver (Fp.: 177 - 179°C).
Ausbeute: 75 % der Theorie.

Beispiel 4

Analog zu Beispiel 3) wird 2,6-Dinitrotoluol-4-(N,N-diisobutyl)sulfonamid hergestellt, indem man an Stelle von Diethylamin. Di-isobutylamin einsetzt.
Silber-graues Pulver (Fp.: 120 - 122°C).
Ausbeute: 50 % der Theorie.

Die Dinitroverbindung wird analog zu Beispiel 2b) zum Diamin hydriert.
Grau-weißes Pulver (Fp.: 155 - 157°C).
Ausbeute: 80 % der Theorie.

Beispiel 5

a) Herstellung von 2,6-Dinitrotoluol-4-(N,N-dimethyl-sulfonamid

Zu einem Gemisch von 28 g (0,1 Mol) 2,6-Dinitrotoluol-4-sulfochlorid und 8,15 g (0,1 Mol) Dimethylaminhydrochlorid in 200 ml Ethanol/Wasser (Volumenverhältnis 1:1) werden bei Raumtemperatur 11,2 g (0,2 Mol) Kaliumhydroxid, gelöst in 50 ml Ethanol/Wasser (Volumenverhältnis 1:1) getropft. Anschließend wird 1 Stunde bei 60°C gerührt. Der Niederschlag wird abgesaugt, mit Wasser gewaschen und getrocknet.
Graugelbes Pulver (Fp.: 138°C).
Ausbeute: 76 % der Theorie.

b) Herstellung von 2,6-Diaminotoluol-4-(N,N-dimethyl)-sulfonamid

41 g 2,6-Dinitrotoulol-4-(N,N-dimethyl)sulfonamid werden in 300 ml Dimethylformamid gelöst und in Gegenwart von 8 g Raney-Nickel unter Druck hydriert.

Das Raney-Nickel wird abfiltriert, das Diamin durch Wasserzugabe ausgefällt, abfiltriert, mehrmals mit Wasser gewaschen und getrocknet.
Fp.: 150 - 152°C.

## Beispiel 6

a) Herstellung von 3,5-Dinitro-4-chlorbenzolsulfochlorid

Zu 250 ml Chlorsulfonsäure werden bei Raumtemperatur innerhalb von 45 Minuten 14 g trockenes K-Salz der 3,5-Dinitro-4-chlorbenzolsulfonsäure gegeben. Anschließend wird 1,5 Stunden bei 100°C gerührt. Das abgekühlte Reaktionsgemisch wird auf ca. 3 l Eiswasser gegeben, der graue Niederschlag wird abfiltriert, mehrmals mit Wasser gewaschen und im Vakuumtrockenschrank bei 50°C getrocknet.
Hellgraues Pulver (Fp.: 83 - 85°C).
Ausbeute: 65 % der Theorie.

b) Herstellung von 3,5-Dinitro-4-chlorbenzol-(N,N-dibutyl)sulfonamid

Zu einer Suspension von 31 g (0,1 Mol) 3,5-Dinitro-4-chlorbenzol-sulfochlorid in 150 ml Ethanol wird bei Raumtemperatur eine Lösung von 27,1 g (0,21 Mol) Dibutylamin in 200 ml Ethanol gegeben. Anschließend

wird 2 Stunden bei 50°C gerührt, der gelbe Niederschlag wird abgesaugt und weiteres Sulfonamid durch Versetzen des Filtrates mit Wasser ausgefällt. Die vereinigten Sulfonamidfraktionen werden mit Wasser gewaschen und im Vakuumtrockenschrank bei 50°C getrocknet.
Gelbes Pulver (Fp.: 97 - 99°C).
Ausbeute: 65 % der Theorie.

| Elementaranalyse: | | gef. | ber. |
|---|---|---|---|
| | C | 42,8 | 42,7 |
| | H | 5,1 | 5,1 |
| | N | 10,6 | 10,7 |
| | S | 8,8 | 8,1 |

c) Herstellung von 3,5-Diamino-4-chlor-benzol-(N,N-dibutyl)sulfonamid

160 g 3,5-Dinitro-4-chlorbenzol-(N,N-dibutyl)sulfonamid werden in 400 ml Ethanol gelöst und in Gegenwart von 30 g Raney-Nickel unter Druck hydriert. Das Raney-Nickel wird abfiltriert und das Filtrat zur Trockne eingeengt.
Der Rückstand wird aus 300 ml Ethanol umkristallisiert.
Grauviolettes Pulver (Fp.: 157°C).
Ausbeute 40 g

| Elementaranalyse: | | gef. | ber. |
|---|---|---|---|
| | C | 50,6 % | 50,4 % |
| | H | 7,0 % | 7,2 % |
| | N | 12,9 % | 12,6 % |
| | Cl | 10,2 % | 10,65 % |
| | S | 9,3 % | 9,6 % |

Patentansprüche

1. Sulfonamidgruppenhaltige aromatische Diamine der Formel

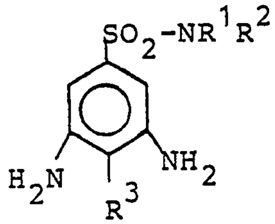

in der

$R^1$ und $R^2$ gleich oder verschieden sind und einen gegebenenfalls verzweigten Alkylrest mit 1 bis 20 C-Atomen oder einen Aryl- oder Aralkylrest mit 6 bis 20 C-Atomen bedeuten und

$R^3$ für ein Wasserstoffatom, einen gegebenenfalls verzweigten Alkylrest mit 1 bis 6 C-Atomen, einen Alkoxyrest mit 1 bis 6 C-Atomen oder ein Halogenatom steht.

2. Diamine nach Anspruch 1, dadurch gekennzeichnet, daß $R^1$ für einen Alkylrest mit 1 bis 6 C-Atomen steht.

3. Diamine nach Anspruch 1 oder 2, dadurch gekennzeichnet, daß $R^3$ für eine Methylgruppe oder für Chlor steht.

4. Verfahren zur Herstellung von Diaminen nach Anspruch 1 bis 3, dadurch gekennzeichnet, daß man ein sekundäres Amin der Formel

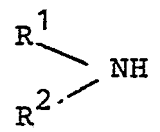

oder dessen Hydrochlorid in Gegenwart von Basen mit einem aromatischen Sulfochlorid der allgemeinen Formel

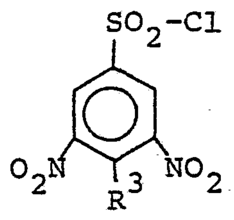

umsetzt und das so erhaltene Dinitrosulfonamid der allgemeinen Formel

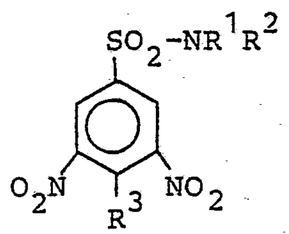

in an sich bekannter Weise zum Diamin reduziert, wobei $R^1$, $R^2$ und $R^3$ die in den Ansprüchen 1 bis 3 angegebene Bedeutung haben.

0037030

Europäisches Patentamt

# EUROPÄISCHER RECHERCHENBERICHT

Nummer der Anmeldung
EP 81 10 2091

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich der maßgeblichen Teile | betrifft Anspruch | |
| X | COLLECTION OF CZECHOSLOVAK CHEMICAL COMMUNICATIONS, Band 36, Nr. 7, 1971<br>P. VETESNIK et al.: "Protonation of polyacid organic bases. VI."<br>Seiten 2500-2506<br>* Seite 2505, Tabel II *<br>-- | 1,2 | C 07 C 143/80//<br>C 08 G 18/38 |
| X | CHEMICAL ABSTRACTS, Band 92, Nr. 6 11. Februar 1980, Zusammenfassung 42598f,Seite 16<br>Columbus, Ohio, US<br>& JP - A - 79 86596 (HITACHI LTD) (10-07-1979)<br>* Zusammenfassung *<br>-- | 1,2 | RECHERCHIERTE SACHGEBIETE (Int. Cl.³)<br>C 07 C 143/80 |
| | FR - A - 2 375 626 (CIBA-GEIGY)<br>* Seite 25 *<br>-- | 1 | |
| | FR - A - 1 253 220 (DU PONT)<br>* Zusammenfassung *<br>-- | 1,4 | |
| | FR - A - 2 133 868 (MERCK)<br>* Patentansprüche *<br>---- | 1,4 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| | Der vorliegende Recherchenbericht wurde für alle Patentansprüche erstellt | | |

| Recherchenort | Abschlußdatum der Recherche | Prüfer |
|---|---|---|
| Den Haag | 03-07-1981 | MOREAU |